Europäisches Patentamt

European Patent Office

Office européen des brevets

(11) Numéro de publication: **0 300 887 B1**

(12) **FASCICULE DE BREVET EUROPEEN**

(45) Date de publication de fascicule du brevet: **27.01.93**   (51) Int. Cl.⁵: **A61K 35/78**

(21) Numéro de dépôt: **88401855.7**

(22) Date de dépôt: **18.07.88**

Le dossier contient des informations techniques présentées postérieurement au dépôt de la demande et ne figurant pas dans le présent fascicule.

(54) **Extrait de cochlospermum planchonii, procédé de préparation et utilisation thérapeutique.**

(30) Priorité: **22.07.87 FR 8710379**

(43) Date de publication de la demande:
**25.01.89 Bulletin  89/04**

(45) Mention de la délivrance du brevet:
**27.01.93 Bulletin  93/04**

(84) Etats contractants désignés:
**AT BE CH DE ES FR GB GR IT LI LU NL SE**

(56) Documents cités:

**REVUE MED. VET., vol. 135, no. 4, 1984; A.SERE et al., pp. 199-209***

(73) Titulaire: **LABORATOIRES DEBAT Société anonyme dite:**
**26, rue Armengaud**
**F-92210 Saint Cloud(FR)**

(72) Inventeur: **Wolga, Jean**
**16, avenue Jeanne d'Arc**
**F-38000 Grenoble(FR)**

(74) Mandataire: **Clisci, Serge et al**
**S.A. FEDIT-LORIOT CONSEILS EN PROPRIETE INDUSTRIELLE 38, avenue Hoche**
**F-75008 Paris(FR)**

## Description

### DOMAINE DE L'INVENTION

La présente invention a trait à un extrait de Cochlospermum planchonii en tant que produit industriel nouveau. Elle concerne également le procédé de préparation et l'utilisation en thérapeutique de cet extrait, notamment en tant qu'agent hépato-protecteur particulièrement intéressant dans le traitement des hépatites virales et fulminantes.

### ART ANTERIEUR

On sait que le Cochlospermum planchonii est un arbuste de la famille des cochlospermacées qui a été notamment décrit par F.R. IRVINE dans l'ouvrage "Woody Plants of Ghana", page 72, Oxford University Press, London 1961. Il pousse enssentiellement en Afrique de l'Ouest, et en particulier au Ghana. On sait que ses racines ont été utilisées en alimentation et médecine indigène.

En tant qu'aliment, les racines qui possèdent une teneur importante en amidon sont préparées bouillies dans de l'eau pour être consommées comme des pommes de terre ou des salsifis.

En médecine indigène, on sait que l'on a préconisé un grand nombre d'indications pour les infusions ou tisanes de racines de Cochlospermum planchonii. Parmi ces indications, il convient de rappeler celle concernant d'une manière générale les ictères de toute nature.

On sait également que les extractions de racines de Cochlospermum planchonii avec des solvants non-polaires ont permis d'isoler des composés qui se sont révélés inactifs en thérapeutique, voir à cet effet les articles de Ivan ADDAE-MENSAH et al., Liebigs Ann. Chem., pages 1284-1287 (1985) et de Hans ACHENBACH, Pure & Appl. Chem., 58 (N°5), pages 653-662 (1986), relatifs à l'isolation de quatre substances dénommées cochlospermines A, B, C et D. L'article de H. ACHENBACH précité enseigne que ces substances, en dépit de leur inactivité biologique, présentent un intérêt théorique dans le cadre de l'identification sans dénaturation ni dégradation de chacun des trois groupes alkyle à chaîne hydrocarbonée linéaire et longue présents dans chaque molécule desdites cochlospermines.

On connait enfin de l'article de A. SERE et al., Revue Med. Vet., 135 (N° 4), pages 199-209, (1984), l'obtention d'un extrait de Cochlospermum tinctorium obtenu par extraction des racines broyées et séchées par de l'eau à température ambiante (15-25°C) pendant 2 jours et susceptible de présenter des propriétés hépato-protectrices.

### BUT ET ORIGINALITE DE L'INVENTION

Suivant l'invention on se propose de préparer un nouvel extrait aqueux de racines de Cochlospermum planchonii, utile en tant qu'agent hépato-protecteur et différent (i) des extraits inactifs obtenus par extraction de racines de Cochlospermum planchonii, avec des solvants non-polaires, et (ii) des infusions et tisanes aqueuses utilisées avec plus ou moins de succès en médecine indigène en tant que moyens anti-ictères, d'une part, et différent de l'extrait aqueux de racines de Cochlospermum tinctorium par la nature de la matière première végétale et les modalités d'extraction, d'autre part.

On a en effet observé suivant l'invention que l'obtention des propriétés hépato-protectrices de l'extrait aqueux de Cochlospermum planchonii dépend en particulier de la température d'extraction. Plus précisément, pour faire passer en solution aqueuse une quantité substantielle de l'ingrédient actif hépato-protecteur, sans altération ou modification essentiellement néfastes vis-à-vis desdites propriétés hépato-protectrices, il faut opérer suivant des conditions opératoires strictes, à savoir, notamment sous la pression atmosphérique (1 atm correspondant approximativement à $10^5$ pascals), à une température d'extraction à l'eau comprise dans la gamme de 56 à 64°C pendant au moins 15 minutes.

Ces conditions strictes ne se présentaient pas lors de la préparation des infusions, macérations et tisanes suivant les techniques usuelles antérieures qui comprennent soit le chauffage de l'eau jusqu'à l'ébullition puis le traitement des racines de la plante avec une eau ayant une température initiale comprise entre 80 et 100°C, soit encore l'extraction de la plante avec de l'eau à la température ambiante (15-25°C).

Par suite, l'effet hépato-protecteur bénéfique de l'extrait aqueux de racines de Cochlospermum planchonii et spécifique que l'on vient de découvrir suivant l'invention ne pouvait pas se manifester en médecine indigène par administration orale desdites infusions, macérations et tisanes.

Un autre but de l'invention est de proposer une nouvelle solution technique permettant de standardiser la préparation de l'extrait hépato-protecteur de racines de Cochlospermum planchonii, de façon que ladite préparation soit reproductible ce qui n'était pas le cas en médecine indigène.

# EP 0 300 887 B1

## RESUME DE L'INVENTION

Le procédé que l'on préconise suivant l'invention pour la préparation d'un extrait de racines de Cochlospermum planchonii, utile notamment en tant qu'agent hépato-protecteur, comprend le traitement desdites racines avec de l'eau. Il est caractérisé en ce que l'on extrait avec de l'eau distillée les racines préalablement séchées et broyées de Cochlospermum planchonii, à une température comprise entre 56°C et 64°C sous une pression d'environ $10^5$ Pa pendant au moins 0,25 h.

De façon pratique ledit procédé comprend les étapes suivantes

a) l'on extrait avec de l'eau distillée les racines préalablement séchées et broyées de Cochlospermum planchonii, à une température comprise entre 56° C et 64°C sous une pression d'environ $10^5$ Pa pendant au moins 0,25 h pour écarter l'insoluble et recueillir la solution aqueuse résultante;

b) on concentre ladite solution aqueuse sous pression réduite à une température inférieure ou égale à 50°C; puis

c) on lyophilise le concentrat ainsi obtenu.

## DESCRIPTION DETAILLEE DE L'INVENTION

Dans le procédé de l'invention la gamme des températures 56-64°C sous une pression de $10^5$ Pa environ est critique. Si l'extraction est effectuée à une température inférieure ou égale à 55°C, on ne recueille pratiquement pas l'ingrédient hépato-protecteur présent dans lesdites racines, d'une part, et si l'extraction est réalisée à une température supérieure ou égale à 65°C, on observe que l'extrait résultant est pratiquement dépourvu d'activité hépato-protectrice (ceci impliquant une destruction ou une altération substantielle de l'ingrédient actif hépato-protecteur), d'autre part. Voir à ce sujet les résultats d'essais comparatifs donnés ci-après.

De façon avantageuse suivant l'invention, sous une pression de $10^5$ Pa environ, la température d'extraction est comprise entre 58 et 62°C et mieux entre 59 et 61°C.

De façon également avantageuse suivant l'invention, on traite 50 g de racines séchées et broyées de Cochlospermum planchonii avec au moins un litre d'eau, la durée de l'extraction étant de 0,5 h.

Après traitement desdites racines séchées et broyées avec de l'eau distillée à une température de 56 à 64°C (de préférence à une température de 58 à 62°C et mieux à une température de 59 à 61°C), on sépare l'insoluble qui est susceptible d'être épuisé par une ou plusieurs autres extractions à l'eau comme indiqué ci-dessus, d'une part, et la solution aqueuse que l'on recueille, d'autre part. Cette séparation est réalisée selon une méthode connue en soi, notamment par filtration ou centrifugation.

Au stade b) on concentre sous pression réduite à une température inférieure ou égale à 50°C la solution aqueuse ainsi recueillie et qui contient l'ingrédient actif hépato-protecteur, dans le cadre d'une extraction unique, ou mieux les solutions aqueuses combinées, dans le cadre d'un épuisement des racines de la plante par des extractions aqueuses successives.

Cette concentration est effectuée de façon avantageuse pour obtenir 1 volume de concentrat aqueux à partir de 4 à 8 volumes d'eau totale utilisée pour l'extraction du stade a), et mieux 1 volume de concentrat pour 5 à 7 volumes d'eau.

La lyophilisation du stade c) est mise en oeuvre de façon classique. On recommande plus particulièrement de congeler le concentrat aqueux obtenu au stade b) à une température de -45°C à -80°C sous pression réduite puis de sublimer l'eau par apport de chaleur lent pour revenir à la pression normale ($10^5$ Pa environ) et à la température ambiante (15-25°C) pendant une durée d'au moins 72 h.

Le meilleur mode de mise en oeuvre du procédé suivant l'invention consiste à effectuer les opérations suivantes :

1°) on extrait avec 1 litre d'eau distillée 50 g de racines séchées et broyées de Cochlospermum planchonii, à une température de 59-61°C sous une pression d'environ $10^5$ Pa pendant 0,5 h, pour recueillir la solution aqueuse;

2°) on extrait deux fois de suite l'insoluble résultant avec de l'eau distillée comme indiqué au stade 1°) ci-dessus, puis rassemble les trois solutions aqueuses qui ont été recueillies et constituent la fraction aqueuse, ladite fraction aqueuse ayant un volume d'environ 3 litres;

3°) on concentre ladite fraction aqueuse sous pression réduite à une température inférieure ou égale à 50° C jusqu'à l'obtention d'un volume de 0,5 litre de concentrat aqueux ; puis

4°) on lyophilise ledit concentrat aqueux.

L'extrait de racines de Cochlospermum planchonii suivant l'invention qui est obtenu grâce au procédé décrit ci-dessus présente dans son spectre UV deux maxima à 210 nm, d'une part, et 265 nm, d'autre part.

Cet extrait, qui est de couleur marron, contient des polyphénols et est dépourvu d'alcaloide et de saponine. Il est totalement soluble dans l'eau et partiellement soluble dans le méthanol et le mélange acétone-eau (6:4) v/v.

D'une manière générale, on obtient 13 à 23 g de lyophilisat à partir de 100 g de racines sèches, soit un rendement de 13 à 23% . Suivant le meilleur mode de mise en oeuvre décrit ci-dessus ledit rendement est de l'ordre de 20 à 23 % .

On préconise suivant l'invention une composition thérapeutique qui est caractérisée en ce qu'elle renferme, en association avec un excipient physiologiquement acceptable, un extrait de racines de Cochlospermum planchonii obtenu selon le procédé décrit ci-dessus.

Bien entendu dans une telle composition ledit extrait intervient en une quantité pharmaceutiquement efficace.

On préconise enfin l'utilisation de l'extrait de racines de Cochlospermum planchonii suivant l'invention pour l'obtention d'un médicament hépato-protecteur destiné à une utilisation en thérapeutique vis-à-vis des hépatites virales et fulminantes.

D'autres avantages et caractéristiques de l'invention seront mieux compris à la lecture de la description qui va suivre d'exemples de préparation et de résultats d'essais pharmacologiques.

Bien entendu, l'ensemble de ces éléments n'est nullement limitatif mais est donné à titre d'illustration.

**Préparation I**

Obtention d'un extrait lyophilisé de racines de Cochlospermum planchonii (Exemple 1, N° de code : D 86.03)

1°) on broye 500 g de racines sèches (ayant une teneur en eau de l'ordre de 5 % en poids par rapport au poids desdites racines) de Cochlospermum planchonii jusqu'à ce qu'on obtienne une poudre ayant une granulométrie moyenne de 6 mm environ, notamment au moyen d'un broyeur à couteaux (par exemple un broyeur commercialisé par la société dite HENRY). Dans un ballon de 20 litres muni d'un agitateur mécanique et équipé d'une enceinte chauffante on met en contact les 500 g de poudre de racines séchées avec 10 litres d'eau distillée. On porte le mélange résultant à 60°C et maintient cette température pendant 0,5 h à la pression atmosphérique (environ $10^5$ Pa). On recueille la solution aqueuse qui contient l'ingrédient hépato-protecteur par filtration, notamment par passage dans un séparateur de laboratoire à bol (par exemple un séparateur commercialisé par la société dite WESTFALIA).

2°) on traite deux fois l'insoluble, obtenu lors de la filtration précitée, dans les mêmes conditions qu'au point 1°) ci-dessus, pour obtenir successivement deux nouvelles solutions aqueuses.

3°) la fraction aqueuse (3 x 10 litres) constituée par les trois solutions aqueuses combinées et qui a un pH de 5, est concentrée sous pression réduite à une température de 45°C jusqu'à un volume de 5 litres de concentrat aqueux, notamment au moyen d'un évaporateur rotatif sous vide.

4°) on lyophilise ledit concentrat ainsi obtenu par congélation, dans des flacons renfermant chacun 200 ml dudit concentrat à -55°C sous vide puis sublimation de l'eau par apport de chaleur en revenant lentement à 25°C et à la pression atmosphérique. La durée de lyophilisation est au total de 6 jours. On obtient ainsi 115 g (rendement : 23 %) d'extrait lyophilisé D 86.03.

**Analyse :**

- l'extrait D 86.03 ne renferme aucun alcaloide (réaction négative au réactif de Draggendorf),
- il ne renferme aucune saponine (réaction négative à l'indice de mousse),
- il contient des polyphénols (coloration gris-noir avec $FeCl_3$), et
- il présente dans son spectre UV deux maxima (à $\lambda$ = 210 nm et $\lambda$ = 265 nm),
- l'étude préliminaire de l'extrait D 86.03 indique la présence d'acide gallique et de ses dérivés (notamment glycosides et esters).

**PREPARATIONS II et III**
(Exemples comparatifs A et B)

En procédant comme indiqué à la préparation I mais en effectuant l'extraction des racines séchées et broyées avec de l'eau distillée à 55°C (par exemple comparatif A) et à 65°C (exemple comparatif B) on obtient deux extraits lyophilisés pour comparaison.

**PREPARATIONS IV et V**

(Exemples 2 et 3)

En procédant comme indiqué à la préparation I mais en effectuant l'extraction des racines séchées et broyées avec de l'eau distillée à 56°C (exemple 2) et à 64°C (exemple 3) on obtient avec un rendement de 17 % des extraits lyophilisés similaires à l'extrait D 86.03 de ladite préparation I.

## ETUDE DES PROPRIETES HEPATO-PROTECTRICES

Comme au cours des essais in vitro l'extrait lyophilisé D 86.03 (Ex 1) a présenté un effet protecteur sur les hépatocytes intoxiqués par le paracétamol, une étude in vivo a été réalisée avec le paracétamol comme agent d'intoxication. Le mécanisme de l'effet hépatotoxique du paracétamol est bien connu : lorsque les voies de glucurono- et de sulfoconjugaison sont saturées, un métabolite hautement réactif est produit par le système des monooxygénases. Ce métabolite se conjugue au glutathion puis après déplétion de celui-ci se fixe de façon covalente aux macromolécules. Il en résulte des altérations membranaires entraînant la libération des enzymes.

La N-acétylcystéine a été choisie comme produit de référence. Cette molécule constitue l'antidote le plus performant lors d'une intoxication par le paracétamol. Son mode d'action exacte n'est pas connu; trois hypothèses ont été émises:

. augmentation du pouvoir de sulfoconjugaison après métabolisme en sulfate inorganique,

. action comme nucléophile (sans transformation en glutathion) et réaction avec le métabolite réactif,

. transformation en L-cystéine et incorporation au pool de gutathion.

Des souris mâles de souche OF1 pesant de 25 à 30 g ont été mises à jeûn sur grille 18 h (instant To - 18 h) avant l'administration du paracétamol.

A l'instant To, le paracétamol (en solution à 12 mg/ml dans du sérum physiologique) a été administré par voie intrapéritonéale à la dose de 400 mg/kg, sous un volume de 0,5 ml.

A l'instant To, on administre également la N-acétylcystéine (en solution à 24 mg/kg dans du sérum physiologique) par voie intrapéritonéale à la dose de 400 mg/kg, sous un volume de 0,5 ml.

L'extrait lyophilisé D 86.03 (exemple I) a été administré par voie intrapéritonéale à la dose de 200 mg/kg à l'instant To-24 h puis à la dose de 200 mg/kg à l'instant To, sous un volume total de 0,5 ml.

Les animaux témoins ont reçu 1 ml de sérum physiologique par voie intrapéritonéale à l'instant To.

Les animaux traités et les animaux témoins ont été sacrifiés à l'instant To + 4 h pour prélèvement du sang (dans des tubes héparinés) et des foies (congelés dans l'azote liquide juste après le sacrifice).

On mesure les valeurs GPT et GOT plasmatiques exprimées en unité internationale par millilitre (UI/l) et la valeur de glutathion hépatique exprimée en nanomoles par gramme (nmole/g). Les résultats obtenus sont consignés dans le tableau I ci-après.

## TABLEAU I

| TRAITEMENT (nombre d'animaux) | GPT (UI/1) | GOT (UI/1) | GLUTATHION HEPATIQUE (nmole/g) |
|---|---|---|---|
| Témoins (n=10) | $52 \pm 10$ | $204 \pm 29$ | $5,24 \pm 0,28$ |
| Paracétamol (n =10) | $634 \pm 161$ | $1\ 510 \pm 395$ | $1,85 \pm 0,36$ |
| Paracétamol + N-Acétyl-cystéine (n = 10) | $248 \pm 38$ | $908 \pm 41$ | $1,84 \pm 0,18$ |
| Paracétamol D 86.03 (n = 10) | $214 \pm 60$ | $491 \pm 100$ | $2,31 \pm 0,35$ |

Notes :
GPT : L-alanine amino transférase.
GOT : 2-oxoglutarate amino transférase

Les résultats du tableau I montrent clairement que

a) l'administration par voie intrapéritonéale d'une dose de 400 mg/kg de paracétamol entraîne une élévation importante des transaminases plasmatiques. Cette libération des transaminases s'accompagne d'une déplétion de glutathion hépatique; et

b) dans les conditions de cette étude, l'extrait D 86.03 présente d'après le dosage des transaminases un effet hépato-protecteur supérieur à celui de la N-acétylcystéine.

Cet effet ne s'accompagne apparemment pas d'une régénération du pool du glutathion hépatique.

Des essais comparatifs avec les extraits des exemples 1 (D 86.03), 2 et 3 selon l'invention et les extraits A et B des préparations II et III, ont été réalisés suivant le même protocole expérimental en ce qui concerne la détermination des GPT et GOT plasmatiques.

Les résultats de ces essais comparatifs consignés dans le tableau II ci-après montrent que les extractions à 55°C et à 65°C ne conduisent pas à l'obtention de l'ingrédient hépato-protecteur.

**TABLEAU II**

| TRAITEMENT (nombre d'animaux) | GPT (UI/1) | GOT (UI/1) |
|---|---|---|
| Témoins (n=10) | 56 ± 5 | 290 ± 36 |
| Paracétamol (n=10) | 680 ± 165 | 1 710 ± 370 |
| Paracétamol + Ex 1 (a) (n=10) | 221 ± 60 | 387 ± 58 |
| Paracétamol + Ex 2 (b) (n=10) | 325 ± 81 | 451 ± 85 |
| Paracétamol + Ex 3 (c) (n=10) | 345 ± 85 | 521 ± 90 |
| Paracétamol + A (d) (n=10) | 650 ± 150 | 1 750 ± 380 |
| Paracétamol + B (e) (n =10) | 685 ± 170 | 1 680 ± 350 |

Notes :

(a) : Ex 1 (D86.03) avec extraction à 60° C

(b) : Ex 2 avec extraction à 56° C

(c) : Ex 3 avec extraction à 64° C

(d) : A avec extraction à 55° C

(e) : B avec extraction à 65° C

Au cours des essais cliniques qui ont été entrepris, l'extrait lyophilisé D 86.03 s'est révélé, par administration orale , particulièrement intéressant chez l'homme adulte vis-à-vis des hépatites virales et fulminantes.

On a ainsi pu traiter avec le D 86.03 et sauver de façon spectaculaire trois patients atteints d'hépatite fulminante avec coma profond déjà installé depuis 1 à 3 jours au moment du début du traitement. L'administration orale de D 86.03 chez ces malades a été effectuée par sonde naso-gastrique. On rappelle que d'une manière constante l'hépatite fulminante évolue vers un coma profond entraînant presque toujours la mort du sujet. La guérison des cas d'hépatite fulminante met en évidence l'intérêt clinique du D 86.03.

Dans l'état actuel des connaissances on pense que l'ingrédient actif hépato-protecteur contenu dans l'extrait suivant l'invention comporte une ou plusieurs substances actives.

**Revendications**
**Revendications pour les Etats contractants suivants : AT, BE, CH, DE, FR, GB, IT, LI, LU, NL, SE**

1. Procédé de préparation d'un extrait de racines de Cochlospermum planchonii, utile notamment en tant qu'agent hépato-protecteur, ledit procédé, qui comprend le traitement desdites racines avec de l'eau, étant caractérisé en ce que l'on extrait avec de l'eau distillée les racines préalablement séchées et broyées de Cochlospermum planchonii, à une température comprise entre 56° C et 64° C sous une pression d'environ $10^5$ Pa pendant au moins 0,25 h.

2. Procédé suivant la revendication 1, caractérisé en ce qu'il comprend les étapes suivantes
   a) l'on extrait avec de l'eau distillée les racines préalablement séchées et broyées de Cochlospermum planchonii, à une température comprise entre 56° C et 64° C sous une pression d'environ $10^5$ Pa pendant au moins 0,25 h pour écarter l'insoluble et recueillir la solution aqueuse résultante;
   b) on concentre ladite solution aqueuse sous pression réduite à une température inférieure ou égale à 50° C; puis
   c) on lyophilise le concentrat ainsi obtenu.

3. Procédé suivant l'une quelconque des revendications 1 et 2, caractérisé en ce que sous une pression de $10^5$ Pa environ, la température d'extraction est comprise entre 58 et 62° C et mieux entre 59 et 61° C.

4. Procédé suivant l'une quelconque des revendications 1 et 2, caractérisé en ce qu'on traite 50 g de racines séchées et broyées de Cochlospermum planchonii avec au moins un litre d'eau, la durée de l'extraction étant de 0,5 h.

5. Procédé suivant la revendication 2, caractérisé en ce que au stade b) la concentration est effectuée de façon à obtenir un volume de concentrat aqueux à partir de 4 à 8 volumes d'eau totale utilisée pour l'extraction du stade a).

6. Procédé suivant la revendication 5, caractérisé en ce que la concentration est effectuée de façon à obtenir 1 volume de concentrat aqueux à partir de 5 à 7 volumes d'eau.

**7.** Procédé suivant l'une quelconque des revendications 1 et 2, caractérisé en ce qu'il comprend les étapes suivantes :

1°) on extrait avec 1 litre d'eau distillée 50 g de racines séchées et broyées de Cochlospermum planchonii, à une température de 59 - 61° C sous une pression d'environ $10^5$ Pa pendant 0,5 h, pour recueillir la solution aqueuse;

2°) on extrait deux fois de suite l'insoluble résultant avec de l'eau distillée comme indiqué au stade 1°) ci-dessus, puis rassemble les trois solutions aqueuses qui ont été recueillies et constituent la fraction aqueuse , ladite fraction aqueuse ayant un volume d'environ 3 litres;

3°) on concentre ladite fraction aqueuse sous pression réduite à une température inférieure ou égale à 50° C jusqu'à l'obtention d'un volume de 0,5 litre de concentrat aqueux ; puis

4°) on lyophilise ledit concentrat aqueux.

**8.** Extrait de racines de Cochlospermum planchonii obtenu suivant le procédé de l'une quelconque des revendications 1 à 7, caractérisé en ce qu'il présente dans son spectre UV deux maxima à 210 nm, d'une part, et à 265 nm, d'autre part.

**9.** Composition thérapeutique caractérisée en ce qu'elle renferme, en association avec un excipient physiologiquement acceptable, un extrait de racines de Cochlospermum planchonii selon la revendication 8.

**10.** Utilisation de l'extrait de racines de Cochlospermum planchonii obtenu selon le procédé de l'une quelconque des revendications 1 à 7, caractérisé en ce que l'on utilise ledit extrait pour l'obtention d'un médicament hépato-protecteur destiné à une utilisation en thérapeutique vis-à-vis des hépatites virales et fulminantes.

**Revendications pour les Etats contractants suivants : ES, GR**

**1.** Procédé de préparation d'un extrait de racines de Cochlospermum planchonii, utile notamment en tant qu'agent hépato-protecteur, ledit procédé, qui comprend le traitement desdites racines avec de l'eau, étant caractérisé en ce que l'on extrait avec de l'eau distillée les racines préalablement séchées et broyées de Cochlospermum planchonii, à une température comprise entre 56° C et 64°C sous une pression d'environ $10^5$ Pa pendant au moins 0,25 h.

**2.** Procédé suivant la revendication 1, caractérisé en ce qu'il comprend les étapes suivantes

a) l'on extrait avec de l'eau distillée les racines préalablement séchées et broyées de Cochlospermum planchonii, à une température comprise entre 56° C et 64°C sous une pression d'environ $10^5$ Pa pendant au moins 0,25 h pour écarter l'insoluble et recueillir la solution aqueuse résultante;

b) on concentre ladite solution aqueuse sous pression réduite à une température inférieure ou égale à 50°C; puis

c) on lyophilise le concentrat ainsi obtenu.

**3.** Procédé suivant l'une quelconque des revendications 1 et 2, caractérisé en ce que sous une pression de $10^5$ Pa environ, la température d'extraction est comprise entre 58 et 62°C et mieux entre 59 et 61°C.

**4.** Procédé suivant l'une quelconque des revendications 1 et 2, caractérisé en ce qu'on traite 50 g de racines séchées et broyées de Cochlospermum planchonii avec au moins un litre d'eau, la durée de l'extraction étant de 0,5 h.

**5.** Procédé suivant la revendication 2, caractérisé en ce que au stade b) la concentration est effectuée de façon à obtenir 1 volume de concentrat aqueux à partir de 4 à 8 volumes d'eau totale utilisée pour l'extraction du stade a).

**6.** Procédé suivant la revendication 5, caractérisé en ce que la concentration est effectuée de façon à obtenir 1 volume de concentrat aqueux à partir de 5 à 7 volumes d'eau.

**7.** Procédé suivant l'une quelconque des revendications 1 et 2, caractérisé en ce qu'il comprend les étapes suivantes :

1°) on extrait avec 1 litre d'eau distillée 50 g de racines séchées et broyées de Cochlospermum planchonii, à une température de 59-61°C sous une pression d'environ $10^5$ Pa pendant 0,5 h, peur recueillir la solution aqueuse;

2°) on extrait deux fois de suite l'insoluble résultant avec de l'eau distillée comme indiqué au stade 1°) ci-dessus, puis rassemble les trois solutions aqueuses qui ont été recueillies et constituent la fraction aqueuse, ladite fraction aqueuse ayant un volume d'environ 3 litres;

3°) on concentre ladite fraction aqueuse sous pression réduite à une température inférieure ou égale à 50°C jusqu'à l'obtention d'un volume de 0,5 litre de concentrat aqueux ; puis

4°) on lyophilise ledit concentrat aqueux.

**Claims**
**Claims for the following Contracting States : AT, BE, CH, DE, FR, GB, IT, LI, LU, NL, SE**

1. A method of preparing an extract of roots of Cochlospermum planchonii, of use inter alia for protecting the liver, the method comprising treatment of the roots with water and being characterised in that previously dried, crushed roots of Cochlospermum planchonii are extracted with distilled water at a temperature between 56°C and 64°C at a pressure of about 105 Pa for a least 0.25 hours.

2. A method according to claim 1, characterised in that it comprises the following steps:
   a) previously dried, crushed roots of Cochlospermum planchonii are extracted with distilled water at a temperature between 56°C and 64°C at a pressure of about $10^5$ Pa for at least 0.25 hours in order to remove the insoluble substance and collect the resulting aqueous solution;
   b) the aqueous solution is concentrated at reduced pressure at a temperature of or below 50°C, and
   c) the resulting concentrate is freeze-dried.

3. A method according to claim 1 or 2, characterised in that at a pressure of about $10^5$ Pa, the extraction temperature is between 58 and 62°C or preferably between 59 and 61°C.

4. A method according to claim 1 or 2, characterised in that 50 g of dried, crushed roots of Cochlospermum planchonii are treated with at least one litre of water, the duration of treatment being 0.5 hours.

5. A method according to claim 2, characterised in that in step b) the method of concentration is such as to obtain one volume of aqueous concentrate from 4 to 8 volumes of total water used for extraction in step a).

6. A method according to claim 5, characterised in that the method of concentration is such as to obtain one volume of aqueous concentrate from 5 to 7 volumes of water.

7. A method according to claim 1 or 2, characterised in that it comprises the following steps:
   1) 50 g of dried, crushed roots of Cochlospermum planchonii are extracted with one litre of distilled water at a temperature of 59 - 61°C and a pressure of about $10^5$ Pa for 0.5 hours, to collect the aqueous solution;
   2) the resulting insoluble substance is extracted twice with distilled water as stated in step 1) hereinbefore, after which the three collected aqueous solutions are combined to form the aqueous fraction, which has a volume of about 3 litres;
   3) the aqueous fraction is concentrated at reduced pressure at a temperature of or below 50°C until a volume of 0.5 litres of aqueous concentrate is obtained, and
   4) the aqueous concentrate is freeze-dried.

8. An extract of roots of Cochlospermum planchonii obtained by the method according to any of claims 1 to 7, characterised in that it has two maxima in its UV spectrum, one at 210 nm and the other at 265 nm.

9. A therapeutic composition, characterised in that it contains an extract of roots of Cochlospermum planchonii according to claim 8, in association with a physiologically acceptable excipient.

10. Use of the extract of roots of Cochlospermum planchonii obtained by the method according to any of claims 1 to 7, characterised in that the extract is used to obtain a liver-protecting drug for therapeutic use against viral or fulminating hepatitis.

**Claims for the following Contracting States : ES, GR**

1.  A method of preparing an extract of roots of Cochlospermum planchonii, of use inter alia for protecting the liver, the method comprising treatment of the roots with water and being characterised in that previously dried, crushed roots of Cochlospermum planchonii are extracted with distilled water at a temperature between 56°C and 64°C at a pressure of about $10^5$ Pa for a least 0.25 hours.

2.  A method according to claim 1, characterised in that it comprises the following steps:
    a) previously dried, crushed roots of Cochlospermum planchonii are extracted with distilled water at a temperature between 56°C and 64°C at a pressure of about $10^5$ Pa for at least 0.25 hours in order to remove the insoluble substance and collect the resulting aqueous solution;
    b) the aqueous solution is concentrated at reduced pressure at a temperature of or below 50°C, and
    c) the resulting concentrate is freeze-dried.

3.  A method according to claim 1 or 2, characterised in that at a pressure of about $10^5$ Pa, the extraction temperature is between 58 and 62°C or preferably between 59 and 61°C.

4.  A method according to claim 1 or 2, characterised in that 50 g of dried, crushed roots of Cochlospermum planchonii are treated with at least one litre of water, the duration of treatment being 0.5 hours.

5.  A method according to claim 2, characterised in that in step b) the method of concentration is such as to obtain one volume of aqueous concentrate from 4 to 8 volumes of total water used for extraction in step a).

6.  A method according to claim 5, characterised in that the method of concentration is such as to obtain one volume of aqueous concentrate from 5 to 7 volumes of water.

7.  A method according to claim 1 or 2, characterised in that it comprises the following steps:
    1) 50 g of dried, crushed roots of Cochlospermum planchonii are extracted with one litre of distilled water at a temperature of 59 - 61°C and a pressure of about $10^5$ Pa for 0.5 hours, to collect the aqueous solution;
    2) the resulting insoluble substance is extracted twice with distilled water as stated in step 1) hereinbefore, after which the three collected aqueous solutions are combined to form the aqueous fraction, which has a volume of about 3 litres;
    3) the aqueous fraction is concentrated at reduced pressure at a temperature of or below 50°C until a volume of 0.5 litres of aqueous concentrate is obtained, and
    4) the aqueous concentrate is freeze-dried.

**Patentansprüche**
**Patentansprüche für folgende Vertragsstaaten : AT, BE, CH, DE, FR, GB, IT, LI, LU, NL, SE**

1.  Verfahren zur Herstellung eines Wurzelextrakts von Cochlospermum planchonii, insbesondere zur Verwendung als Leberschutzmittel, das die Behandlung dieser Wurzeln mit Wasser einschließt und dadurch gekennzeichnet ist, daß man die zuvor getrockneten und zerstoßenen Wurzeln von Cochlospermum planchonii bei einer Temperatur zwischen 56°C und 64°C unter einem Druck von etwa $10^5$ Pa über wenigstens 0,25 h extrahiert.

2.  Verfahren nach Anspruch 1, dadurch gekennzeichnet, daß es die folgenden Schritte einschließt:
    a) Man extrahiert mit destilliertem Wasser die zuvor getrockneten und zerstoßenen Wurzeln von Cochlospermum planchonii bei einer Temperatur zwischen 56°C und 64°C unter einem Druck von etwa $10^5$ Pa über wenigstens 0,25 h, um das Unlösliche abzuscheiden und die resultierende wäßrige Lösung zu sammeln;
    b) man konzentriert die wäßrige Lösung unter vermindertem Druck bei einer Temperatur unterhalb oder gleich 50°C; dann
    c) gefriertrocknet man das das so erhaltene Konzentrat.

**3.** Verfahren nach einem der Ansprüche 1 und 2, dadurch gekennzeichnet, daß die Extraktionstemperatur, bei einem Druck von etwa $10^5$ Pa, zwischen 58 und 62°C, besser zwischen 59 und 61°C, liegt.

**4.** Verfahren nach einem der Ansprüche 1 und 2, dadurch gekennzeichnet, daß man 50 g der getrockneten und zerstoßenen Wurzeln von Cochlospermum planchonii mit wenigstens einem Liter Wasser behandelt, wobei die Extraktionsdauer 0,5 h beträgt.

**5.** Verfahren nach Anspruch 2, dadurch gekennzeichnet, daß in Stufe b) die Konzentration auf eine Weise bewirkt wird, daß ein Volumen wäßriges Konzentrat ausgehend von 4 bis 8 Volumina insgesamt für die Extraktion in Stufe a) verwandtes Wasser erhalten wird.

**6.** Verfahren nach Anspruch 5, dadurch gekennzeichnet, daß die Konzentration auf eine Weise bewirkt wird, daß ein Volumen wäßriges Konzentrat ausgehend von 5 bis 7 Volumina Wasser erhalten wird.

**7.** Verfahren nach einem der Ansprüche 1 und 2, dadurch gekennzeichnet, daß es die folgenden Schritte umfaßt:
1) Man extrahiert mit einem Liter destilliertem Wasser 50 g getrocknete und zerstoßene Wurzeln von Cochlospermum planchonii bei einer Temperatur von 59 bis 61°C unter einem Druck von etwa $10^5$ Pa über 0,5 h, um eine wäßrige Lösung zu sammeln;
2) man extrahiert anschließend zweimal das resultierende Unlösliche mit destilliertem Wasser, wie für Stufe 1.) vorstehend angegeben und vereinigt die drei wäßrigen Lösungen, die erhalten wurden und die wäßrige Fraktion bilden, wobei die wäßrige Fraktion ein Volumen von ungefähr 3 l ausmacht;
3) man konzentriert die wäßrige Fraktion unter vermindertem Druck bei einer Temperatur von weniger als oder gleich 50°C bis zum Erhalt eines Volumens von 0,5 l wäßrigem Konzentrat; danach
4) gefriertrocknet man das wäßrige Konzentrat.

**8.** Wurzelextrakt aus Cochlospermum planchonii, erhalten nach dem Verfahren eines der Ansprüche 1 bis 7, dadurch gekennzeichnet, daß es in seinem UV-Spektrum 2 Maxima bei einerseits 210 nm und andererseits 265 nm zeigt.

**9.** Therapeutische Zusammensetzung, dadurch gekennzeichnet, daß sie zusammen mit einem physiologisch annehmbaren Excipienten einen Wurzelextrakt von Cochlospermum planchonii nach Anspruch 8 einschließt.

**10.** Verwendung des nach dem Verfahren nach einem der Ansprüche 1 bis 7 erhaltenen Wurzelextrakts von Cochlospermum planchonii, dadurch gekennzeichnet, daß man diesen Extrakt für den Erhalt eines Leberschutzmedikaments verwendet, das zur Verwendung in der Therapie von Virus- und akuter Hepatitis.

**Patentansprüche für folgende Vertragsstaaten : ES, GR**

**1.** Verfahren zur Herstellung eines Wurzelextrakts von Cochlospermum planchonii, insbesondere zur Verwendung als Leberschutzmittel, das die Behandlung dieser Wurzeln mit Wasser einschließt und dadurch gekennzeichnet ist, daß man die zuvor getrockneten und zerstoßenen Wurzeln von Cochlospermum planchonii bei einer Temperatur zwischen 56°C und 64°C unter einem Druck von etwa $10^5$ Pa über wenigstens 0,25 h extrahiert.

**2.** Verfahren nach Anspruch 1, dadurch gekennzeichnet, daß es die folgenden Schritte einschließt:
a) Man extrahiert mit destilliertem Wasser die zuvor getrockneten und zerstoßenen Wurzeln von Cochlospermum planchonii bei einer Temperatur zwischen 56°C und 64°C unter einem Druck von etwa $10^5$ Pa über wenigstens 0,25 h, um das Unlösliche abzuscheiden und die resultierende wäßrige Lösung zu sammeln;
b) man konzentriert die wäßrige Lösung unter vermindertem Druck bei einer Temperatur unterhalb oder gleich 50°C; dann
c) gefriertrocknet man das so erhaltene Konzentrat.

**3.** Verfahren nach einem der Ansprüche 1 und 2, dadurch gekennzeichnet, daß die Extraktionstemperatur, bei einem Druck von etwa $10^5$ Pa, zwischen 58 und 62°C, besser zwischen 59 und 61°C, liegt.

**4.** Verfahren nach einem der Ansprüche 1 und 2, dadurch gekennzeichnet, daß man 50 g der getrockneten und zerstoßenen Wurzeln von Cochlospermum planchonii mit wenigstens einem Liter Wasser behandelt, wobei die Extraktionsdauer 0,5 h beträgt.

**5.** Verfahren nach Anspruch 2, dadurch gekennzeichnet, daß in Stufe b) die Konzentration auf eine Weise bewirkt wird, daß ein Volumen wäßriges Konzentrat ausgehend von 4 bis 8 Volumina insgesamt für die Extraktion in Stufe a) verwandtes Wasser erhalten wird.

**6.** Verfahren nach Anspruch 5, dadurch gekennzeichnet, daß die Konzentration auf eine Weise bewirkt wird, daß ein Volumen wäßriges Konzentrat ausgehend von 5 bis 7 Volumina Wasser erhalten wird.

**7.** Verfahren nach einem der Ansprüche 1 und 2, dadurch gekennzeichnet, daß es die folgenden Schritte umfaßt:
1) Man extrahiert mit einem Liter destilliertem Wasser 50 g getrocknete und zerstoßene Wurzeln von Cochlospermum planchonii bei einer Temperatur von 59 bis 61°C unter einem Druck von etwa $10^5$ Pa über 0,5 h, um eine wäßrige Lösung zu sammeln;
2) man extrahiert anschließend zweimal das resultierende Unlösliche mit destilliertem Wasser, wie für Stufe 1.) vorstehend angegeben und vereinigt die drei wäßrigen Lösungen, die erhalten wurden und die wäßrige Fraktion bilden, wobei die wäßrige Fraktion ein Volumen von ungefähr 3 l ausmacht;
3) man konzentriert die wäßrige Fraktion unter vermindertem Druck bei einer Temperatur von weniger als oder gleich 50°C bis zum Erhalt eines Volumens von 0,5 l wäßrigem Konzentrat; danach
4) gefriertrocknet man das wäßrige Konzentrat.